# FASCICULE DE BREVET EUROPEEN

(11) **EP 1 713 414 B1**
(45) Date de publication et mention de la délivrance du brevet: **31.03.2010**
(21) Numéro de dépôt: 05717592.9
(22) Date de dépôt: 09.02.2005
(51) Int. Cl.: A61F 2/04

(54) **PROTHESE TUBULAIRE**
RÖHRENFÖRMIGE PROTHESE
TUBULAR PROSTHESIS

(30) Priorité: 11.02.2004 FR 0401366
(43) Date de publication de la demande: 25.10.2006
(73) Titulaire: Laboratoires Perouse, 60173 Ivry Le Temple (FR)
(72) Inventeur: PEROUSE, Eric, F-75016 PARIS (FR)
(74) Mandataire: Domenego, Bertrand
(86) Numéro de dépôt international: PCT/FR2005/000291
(87) Numéro de publication internationale: WO 2005/079705

(56) Documents cités:
- WO-A-00/07506
- WO-A-02/085254
- US-A- 5 720 755
- US-A1- 2001 014 822
- US-A1- 2002 123 790

## Description

La présente invention concerne une prothèse tubulaire, du type déformable radialement comprenant un treillis déformable entre un état rétracté de diamètre réduit et un état dilaté de diamètre plus grand.

Il est connu, pour différents types de traitement, de mettre en place une prothèse tubulaire à l'intérieur d'un vaisseau sanguin, qu'il s'agisse d'une veine ou d'une artère. De telles prothèses tubulaires sont généralement désignées par le terme "stent".

La prothèse est amenée à l'intérieur du vaisseau alors qu'elle est dans son état rétracté puis pour sa mise en place, la prothèse est dilatée pour s'appliquer contre la surface intérieure du vaisseau. Cette dilatation s'effectue soit automatiquement du fait de l'élasticité propre au treillis de la prothèse, soit sous l'action du gonflement d'un ballonnet intérieur, provoquant une déformation plastique du matériau constituant le treillis.

La retenue axiale de la prothèse dans le vaisseau, c'est-à-dire suivant la longueur du faisceau est délicate à assurer, et la prothèse risque de se déplacer suivant la longueur du faisceau sous l'action de l'écoulement du flux sanguin. De plus, la prothèse risque de ne pas être appliquée exactement à la surface du vaisseau sanguin du fait de sa section irrégulière.

Pour éviter ce déplacement, il est connu que l'extrémité du treillis métallique présente des crans faisant saillie vers l'extérieur et propres à pénétrer dans la paroi du vaisseau afin d'immobiliser axialement la prothèse.

Il est également connu de solidariser la prothèse à la paroi du vaisseau, par exemple par mise en place d'agrafes rapportées après mise en place de la prothèse.

Le document US 2001/014822 décrit la prothèse du préambule de la revendication 1.

Ces moyens de fixation sont peu fiables ou délicats à mettre en place.

L'invention a pour but de proposer une prothèse tubulaire permettant un positionnement fiable et dont la mise en place est relativement aisée.

A cet effet, l'invention a pour objet une prothèse tubulaire du type précité, caractérisée en ce qu'elle comporte au moins deux crochets extérieurs délimitant entre eux une pince d'accrochage dans un tissu extérieur, les deux crochets étant portés par le treillis et déplaçables entre une position écartée dans laquelle la pince est ouverte et une position rapprochée dans laquelle la pince est fermée.

Suivant des modes particuliers de réalisation, la prothèse tubulaire comporte l'une ou plusieurs des caractéristiques suivantes :
- chaque crochet est lié au treillis depuis une extrémité de liaison et les crochets d'une même pince sont mobiles l'un par rapport à l'autre lors de la déformation de la prothèse ;
- le treillis comporte des fils entrecroisés pour former un maillage de quadrilatères déformables, et chaque crochet est lié au treillis dans un coin du quadrilatère ;
- chaque crochet est soudé au treillis à son extrémité de liaison ;
- chaque crochet est prolongé à son extrémité de liaison par un brin torsadé autour du treillis ;
- chaque crochet d'une même pince présente à son extrémité d'accrochage une forme de crosse, les deux crochets se chevauchant au moins partiellement pour former ladite pince ;
- chaque crochet présente une forme de lame sensiblement rectiligne, les deux crochets s'étendant en regard l'un de l'autre et à l'écart l'un de l'autre lorsque la pince est ouverte ; et
- le treillis est déformable élastiquement vers sa position dilatée.

L'invention est également relative à un nécessaire de traitement d'un vaisseau sanguin, caractérisé en ce qu'il comporte :
- une prothèse telle que décrite ci-dessus ;
- des moyens de retenue du treillis rétracté dans la région de la ou chaque pince ;
- un tube de largage du treillis délimitant un conduit de confinement de la prothèse dans son état rétracté.

Suivant un mode particulier de réalisation, le conduit de confinement du tube de largage comporte des canaux longitudinaux de réception des crochets.

L'invention sera mieux comprise à la lecture de la description qui va suivre, donnée uniquement à titre d'exemple et faite en se référant aux dessins, sur lesquels :
- la figure 1 est une vue en perspective d'une prothèse tubulaire à l'état dilaté;
- la figure 2 est une vue en section à plus grande échelle de la prothèse à l'état dilaté dans une région comportant une pince ;
- la figure 3A est une vue en section transversale de la prothèse à l'état rétracté ;
- la figure 3B est une vue en perspective de la prothèse en cours d'implantation dans son état rétracté de la figure 3A ;
- la figure 3C est une vue en section à plus grande échelle de la prothèse dans son état rétracté dans une région comportant une pince en position ouverte ;
- la figure 4A est une vue identique à celle de la figure 3A de la prothèse dilatée avant accrochage ;
- la figure 4B est une vue en perspective de la prothèse dilatée avant accrochage ;
- la figure 5A est une vue identique à la figure 3A de la prothèse dilatée et accrochée après retrait des organes de retenue des pinces ;
- la figure 5B est une vue en perspective de la prothèse dilatée après retrait des organes de retenue des pinces ; et
- les figures 6 et 7 sont des vues en perspective et en élévation de variantes de réalisation de la prothèse selon l'invention.

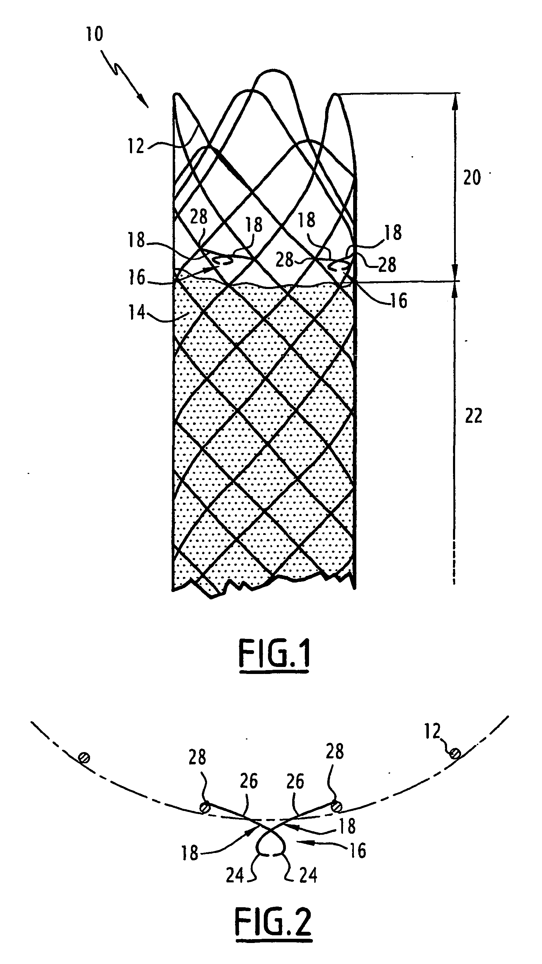

La prothèse tubulaire 10 représentée sur la figure 1 est destinée à être mise en place dans un vaisseau sanguin. Elle comporte un treillis tubulaire 12 noyé, sur l'essentiel de la longueur de la prothèse, dans un film 14. La prothèse comporte en outre, au voisinage de l'une de ses extrémités, trois pinces 16 régulièrement réparties angulairement à sa périphérie.

Chaque pince 16 est formée de deux crochets 18 portés par le treillis métallique 12. Ces crochets sont déplaçables l'un par rapport à l'autre entre une position écartée dans laquelle la pince est ouverte et une position rapprochée dans laquelle la pince est fermée.

Les pinces 16 sont prévues sur un tronçon d'extrémité 20 de la prothèse dépourvu de film 14, le treillis 12 n'étant ainsi pas recouvert dans cette région. En revanche, dans le tronçon principal noté 22 de la prothèse, le treillis 12 est noyé dans le film 14.

Le treillis 12 est constitué d'acier inoxydable de qualité biocompatible. Il est réalisé par exemple par tressage ou tricotage d'un fil, déploiement axial d'un tube, ou par toute autre technique appropriée.

Dans le mode de réalisation illustré à la figure 1, le treillis 12 est constitué de deux faisceaux de fils enroulés en hélice en des sens opposés, les fils d'un même faisceau s'étendant généralement parallèlement les uns aux autres et transversalement aux fils du faisceau de fils enroulés en sens inverse.

Les fils des deux faisceaux d'enroulements opposés se croisent alternativement au-dessus et en dessous.

Le treillis 12 est de préférence élastiquement déformable par expansion radiale, entre un état rétracté de petit diamètre et un état dilaté de plus grand diamètre.

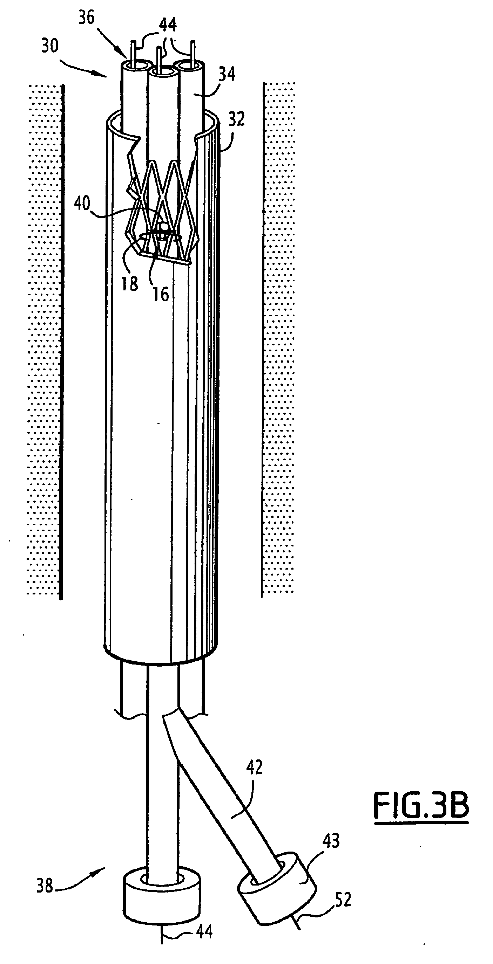

Dans son état dilaté, illustré sur la figure 1, les mailles du treillis forment des losanges généralement allongés dans le sens circonférentiel. Au contraire, et comme illustré sur la figure 3B, à l'état rétracté de la prothèse, les mailles forment des losanges allongés parallèlement à l'axe de la prothèse.

En variante, la prothèse est plastiquement déformable, c'est-à-dire que le treillis possède une première forme stable de petit diamètre et une seconde forme stable de diamètre agrandi.

Sur le tronçon 22, le treillis 12 est entièrement noyé dans le film 14. Ce film est formé d'une matière extensible et étanche aux liquides qui emplit les mailles.

L'extensibilité de cette matière est suffisante pour que le film 14 suive la déformation du treillis de son état rétracté à son état dilaté sans déchirure ni décollement, malgré la déformation des mailles et du treillis. Des matières appropriées sont un élastomère biocompatible qui peut être un caoutchouc naturel ou synthétique ou bien un polymère biocompatible tel qu'un polyuréthanne.

L'enrobage du treillis 12 par le film 14 est obtenu par exemple par une technique de co-extrusion ou de trempage, après dégraissage du métal et son traitement par une substance primaire d'adhérence.

Comme illustré sur la figure 2, les crochets 18 sont formés chacun d'un tronçon métallique filiforme dont une extrémité libre est recourbée vers l'extérieur pour former une crosse 24. Cette crosse 24 prolonge un tronçon rectiligne 26. Les crochets 18 se chevauchent au moins partiellement pour former une pince 16.

A son extrémité opposée à la crosse 24, chaque tronçon 26 est soudé au treillis métallique 12 dans les coins opposés d'une maille par des points de soudure 28 visible sur la figure 1. Les crosses 24 font saillie vers l'extérieur par rapport au tronçon tubulaire délimité par le treillis 12 et leurs extrémités recourbées s'étendent, au repos, dans un plan transversal à la prothèse tubulaire, c'est-à-dire perpendiculaire à son axe général, comme illustré sur la figure 2.

Le diamètre des crosses 24 des crochets est compris entre 0,5 mm et 4 mm, alors que la longueur du bras 26 est comprise entre 3 mm et 12 mm. De préférence, le diamètre des crosses 24 formant l'extrémité recourbée des crochets est compris entre un quart et un huitième de la longueur du bras 26 du crochet.

La longueur des bras 26 est telle que, dans l'état dilaté de la prothèse illustré sur la figure 1, les deux crosses des crochets 18 sont rapprochées et délimitent ensemble une boucle fermée ou pratiquement fermée.

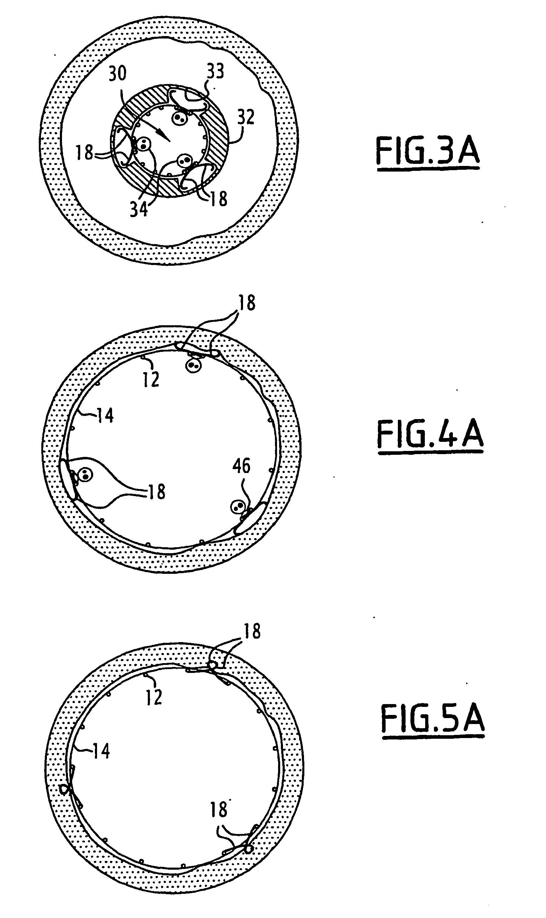

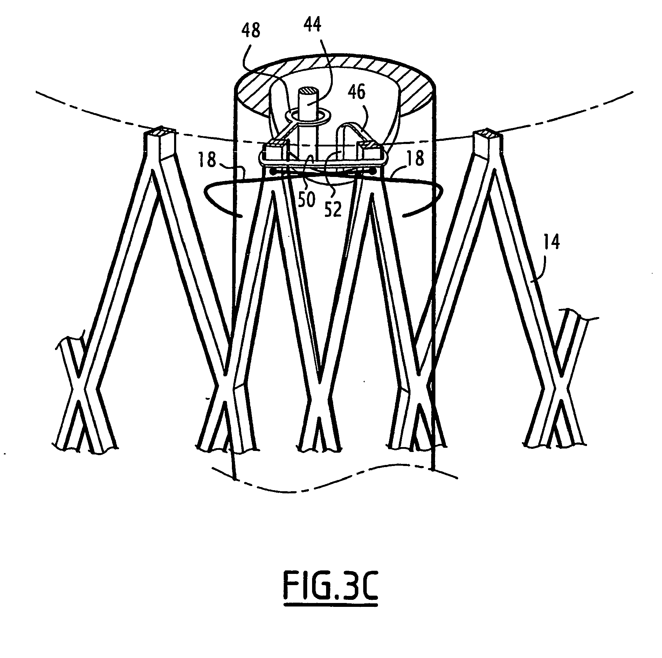

Initialement, et comme illustré sur les figures 3A, 3B et 3C, la prothèse est associée à des moyens 30 de retenue des pinces 16 dans leur position ouverte.

En outre, la prothèse dont les pinces sont maintenues ouvertes est reçue, comme connu en soi, dans un tube de largage 32 à l'intérieur duquel la prothèse est confinée, dans son état rétracté.

Avantageusement, le conduit intérieur du tube 32 présente longitudinalement des canaux 33 de réception des extrémités des crochets 18 faisant saillie par rapport à la surface généralement tubulaire du treillis métallique.

Comme illustré sur les figures 3B et 3C, chaque moyen de retenue d'une pince ouverte comporte un tube flexible 34 formé par exemple en PEEK. Ce tube 34 s'étend longitudinalement entre une extrémité distale 36 destinée à être reçue dans le vaisseau sanguin et une proximité proximale 38 destinée à être accessible par le chirurgien hors du corps du patient. Ainsi, le tube 34 a par exemple une longueur de un mètre.

Une ouverture de retenue 40 est ménagée latéralement dans le tube 34 généralement en regard de la pince 16 associée. Le tube 34 est équipé par ailleurs, au voisinage de son extrémité proximale 38, d'un embranchement latéral creux 42 équipé d'une bague 43 de blocage axial d'un fil coulissant.

Les moyens de retenue libérables 30 comprennent en outre une tige de retenue 44 engagée axialement dans le tube 34, et un fil de retenue 46 ceinturant la maille de la prothèse portant la pince 16.

La tige de retenue 44 s'étend d'un bout à l'autre du tube 34. Elle fait saillie hors du tube à l'extrémité proximale 38.

Cette tige est mobile dans le tube 34 entre une position de retenue dans laquelle la tige est en regard de l'ouverture 40 et une position de libération dans laquelle la tige 44 est à l'écart de l'ouverture 40 et est décalée vers l'extrémité proximale du tube 34.

Le fil de retenue 46 comprend un brin unique qui comporte à une extrémité un passant 48, une boucle de serrage 50 et un tronçon de commande 52 qui s'étend suivant toute la longueur du tube 34 de l'ouverture 40 à l'embranchement 42 hors duquel il fait saillie après avoir traversé la bague de blocage 43.

Le passant d'extrémité 48 est formé d'une boucle fermée de petit diamètre dans laquelle est engagée initialement la tige 44 lorsque celle-ci est dans sa position de retenue. La boucle de serrage 50 est formée par un tronçon du brin, engagée de manière coulissante au travers de deux mailles du treillis adjacentes à la maille portant la pince 16.

La boucle de serrage traverse l'ouverture 40 pour rejoindre le passant 48 à une extrémité et le tronçon de commande 52 à son autre extrémité. La longueur active de la boucle de serrage 50 est variable en fonction de la traction exercée sur le tronçon de commande 52, de sorte qu'elle contrôle la forme de la maille portant la pince 16, comme cela sera exposé dans la suite.

Initialement, avant mise en place, la prothèse est disposée dans le tube de largage 32 et les tronçons de commande 52 des moyens de retenue des pinces sont tendus, de sorte que les pinces sont maintenues ouvertes, comme illustré sur la figure 3B. En effet, dans cette position, la boucle de serrage 50 resserre la maille portant la pince, de sorte que le losange définissant la maille est allongé suivant sa diagonale parallèle à l'axe de la prothèse.

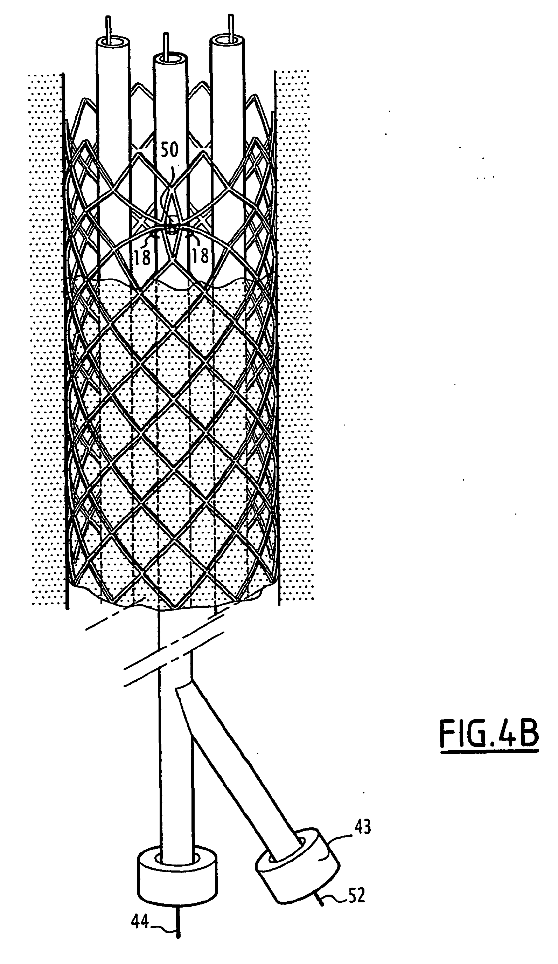

Pour la mise en place de la prothèse, celle-ci est introduite avec le tube 32 jusqu'à la zone de largage puis le tube 32 est retiré libérant ainsi la prothèse. Celle-ci se dilate et est alors plaquée contre la surface intérieure du vaisseau sanguin, comme illustré sur les figures 4A et 4B.

Lors de cette dilatation, les mailles du treillis de la prothèse s'étendent grâce à l'élasticité du treillis suivant la diagonale périphérique de la prothèse permettant ainsi une augmentation du diamètre de la prothèse. En revanche, les mailles portant une pince restent contractées, comme illustré sur la figure 4B du fait de la boucle de serrage 50. Ainsi, les pinces 16 sont accostées contre la surface du vaisseau sanguin alors que celles-ci sont encore en position ouverte.

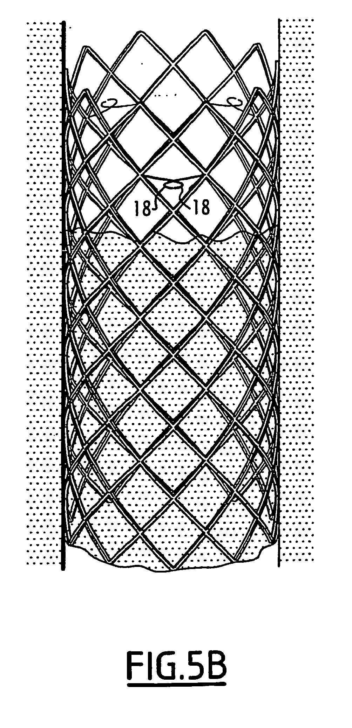

En agissant sur la bague de blocage 43, le praticien procède alors à la libération des fils de retenue afin d'autoriser la déformation élastique des mailles portant les pinces, et ainsi le rapprochement des deux crochets opposés, provoquant la fermeture de la pince et la pénétration des crochets dans la paroi délimitant le vaisseau sanguin, comme illustré sur les figures 5A et 5B.

Après relâchement des fils de retenue 46, la tige 44 est amenée en position de libération, de sorte que le passant 48 est libéré de la tige 44. Le praticien tire alors sur le tronçon de commande 52 permettant que le fil de retenue s'échappe du treillis métallique, en circulant au travers des deux mailles adjacentes à la maille portant la pince.

Ainsi, les moyens de retenue de la pince étant rendus indépendants de la prothèse, ceux-ci peuvent être extraits par voie endoluminale.

On comprend qu'une telle prothèse est retenue efficacement contre la surface interne du vaisseau par la présence des pinces qui sont maintenues élastiquement en position fermée sous l'action de la prothèse. De plus, les pinces étant refermées simultanément à la mise en place de la prothèse, l'installation d'une telle prothèse est relativement aisée.

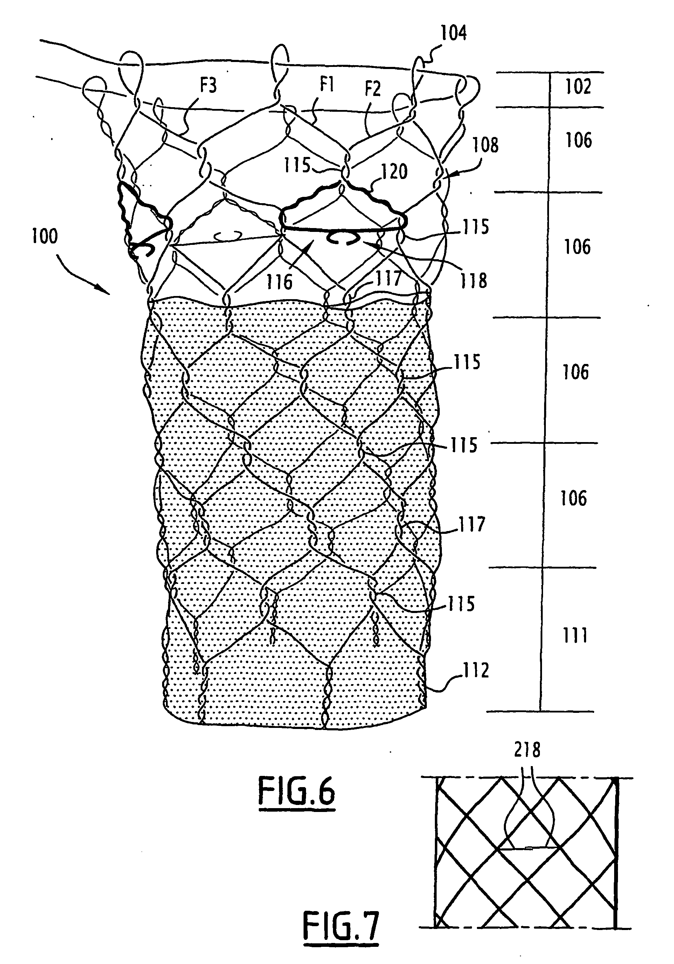

Sur la figure 6 est représentée une variante de réalisation d'une prothèse selon l'invention.

La prothèse vasculaire représentée sur la figure 6 comprend un treillis 100 lui-même formé par huit fils métalliques élastiques, tels que les fils F1, F2 et F3, torsadés ensemble d'une manière qui sera détaillée ci-dessous. Ces fils définissent, sur la longueur du treillis, plusieurs régions successives qui sont, de haut en bas sur la figure 6 :
- une région d'extrémité 102 à huit boucles 104 ;
- des régions successives 106 qui présentent chacune des couronnes périphériques de noeuds torsadés 108 ;
- une région d'extrémité 111 à torsades d'extrémité 112.

En considérant le fil F1 :
- la boucle 104 est constituée par la torsade du fil D1 sur lui-même, sur au moins un demi-tour ;
- chaque noeud 108 est constitué par la torsade du fil F1 avec un fil adjacent tel que F2, F3 sur un tour ou plus.

Ainsi, de part et d'autre de chaque noeud dit à torsade double et noté 115 où le fil est torsadé sur un nombre pair de demi-tours notamment égal à deux, chaque brin de fil qui forme le noeud s'étend suivant deux directions parallèles l'une à l'autre et proches l'une de l'autre.

Au contraire, en ce qui concerne les noeuds dits à torsade triple 117 où le fil est torsadé sur un nombre impair de demi-tours notamment égal à trois, chaque fil en part suivant deux directions qui forment entre elles un angle très inférieur à 180°, par exemple un angle droit ou un angle aigu comme représenté, pour constituer deux côtés adjacents d'une maille du treillis.

Comme indiqué sur la figure 6, dans l'exemple représenté, le fil F1 part d'une torsade 112 puis forme successivement un noeud 115, un noeud 117, un noeud 115, un autre noeud 115, un noeud 117, un noeud 115, un autre noeud 115, une boucle 113, un noeud 115, un autre noeud 115, un noeud 117, un noeud 115, un autre noeud 115, un noeud 117, un noeud 115 et une autre torsade 112.

Dans ce mode de réalisation, chaque pince notée 116 est formée d'un unique fil métallique 120 dont la partie courante est engagée et torsadée autour des fils délimitant le treillis, et dont les deux extrémités libres sont recourbées vers l'extérieur pour former des crochets 118.

Plus précisément, le fil 120 est torsadé depuis un noeud 115 autour de deux brins divergents angulairement décalés. Chaque branche du fil 120 est ensuite torsadée avec le noeud torsadé suivant et s'étend ensuite transversalement suivant un diamètre de la maille, les deux bras se rejoignant en s'étendant parallèlement l'un à l'autre à une extrémité recourbée.

Ainsi, comme dans le mode de réalisation précédent, la déformation de la maille portant la pince provoque l'ouverture ou la fermeture de la pince, les deux bras à extrémité recourbée se déplaçant l'un par rapport à l'autre.

Dans le mode de réalisation de la figure 7, les crochets 218 sont formés par de simples tiges généralement rectilignes dont une extrémité est solidarisée à l'extrémité d'une maille et dont l'autre extrémité fait légèrement saillie vers l'extérieur par rapport à la surface extérieure généralement cylindrique du treillis.

Dans ce mode de réalisation, lorsque les mailles sont ouvertes, les extrémités pointues des deux crochets 218 se chevauchent. En revanche, lorsque les deux mailles adjacentes disposées de part et d'autre de la maille portant la pince sont fermées, les crochets sont écartés l'un de l'autre.

Pour la mise en place d'une telle prothèse, deux moyens de retenue fermés des mailles sont nécessaires pour une même pince. Initialement, ils sont appliqués sur les deux mailles adjacentes afin de permettre de maintenir la pince ouverte. Après que la prothèse a été correctement positionnée, les deux mailles adjacentes sont libérées, ce qui conduit à une fermeture de la pince et une pénétration des deux crochets dans la paroi du vaisseau sanguin.

## Revendications

1. Prothèse tubulaire (10) déformable radialement comprenant un treillis (12) déformable entre un état rétracté de diamètre réduit et un état dilaté de diamètre plus grand, portés par le treillis (12) et au moins deux crochets extérieurs (18 ; 118 ; 218) **caractérisé en ce que**, les deux crochets (18 ; 118 ; 218) délimitent entre eux une pince (16) d'accrochage dans un tissu extérieur et sont déplaçables entre une position écartée dans laquelle la pince (18 ; 118 ; 218) est ouverte et une position rapprochée dans laquelle la pince (16) est fermée.

2. Prothèse selon la revendication 1, **caractérisée en ce que** chaque crochet (18 ; 118 ; 218) est lié au treillis (12) depuis une extrémité de liaison et les crochets (18 ; 118 ; 218) d'une même pince (16) sont mobiles l'un par rapport à l'autre lors de la déformation de la prothèse.

3. Prothèse selon la revendication 2, **caractérisée en ce que** le treillis (12) comporte des fils entrecroisés pour former un maillage de quadrilatères déformables, et **en ce que** chaque crochet (18 ; 118 ; 218) est lié au treillis dans un coin du quadrilatère.

4. Prothèse selon la revendication 2 ou 3, **caractérisée en ce que** chaque crochet (18 ; 218) est soudé au treillis (12) à son extrémité de liaison.

5. Prothèse selon la revendication 2 ou 3, **caractérisée en ce que** chaque crochet (118) est prolongé à son extrémité de liaison par un brin tor sadé (120) autour du treillis (12).

6. Prothèse selon l'une quelconque des revendications précédentes **caractérisée en ce que** chaque crochet (18) d'une même pince présente ; son extrémité d'accrochage une forme de crosse (24), les deux crochet (18) se chevauchant au moins partiellement pour former ladite pince (16).

7. Prothèse selon l'une quelconque des revendications 1 à 5, **caractérisée en ce que** chaque crochet (218) présente une forme de lame sens blement rectiligne, les deux crochets (218) s'étendant en regard l'un de l'autre et à l'écart l'un de l'autre lorsque la pince est ouverte.

8. Prothèse selon l'une quelconque des revendications précédentes, **caractérisée en ce que** le treillis (12) est déformable élastiquement vers sa position dilatée.

9. Nécessaire de traitement d'un vaisseau sanguin, **caractérisé en ce qu'**il comporte :
- une prothèse selon les revendications 2 et 8 ;
- des moyens (30) de retenue du treillis rétracté dans la région de la ou chaque pince ;
- un tube (32) de largage du treillis délimitant un conduit de confinement de la prothèse dans son état rétracté.

10. Nécessaire selon la revendication 9, **caractérisé en ce que** ledit conduit de confinement du tube de largage (32) comporte des canaux longitudinaux (33) de réception des crochets (18).

## Claims

1. Radially deformable tubular prosthesis (10) comprising a mesh (12), deformable between a small diameter retracted state and a larger diameter expanded state, and at least two external hooks (18, 118, 218) carried by the mesh (12), **characterised in that** the two hooks (18, 118, 218) define between them a clip (16) for catching in external tissue and are movable between a spaced-apart position in which the hook (18, 118, 218) is open and a close-together position in which the clip (16) is closed.

2. Prosthesis according to Claim 1, **characterised in that** each hook (18, 118, 218) is connected to the mesh (12) from a connection end and the hooks (18, 118, 218) of a single clip (16) are movable with respect to each other during deformation of the prosthesis.

3. Prosthesis according to Claim 2, **characterised in that** the mesh (12) comprises wires interlaced to form a mesh-work of deformable quadrilaterals and **in that** each hook (18, 118, 218) is connected to the mesh in a corner of the quadrilateral.

4. Prosthesis according to Claim 2 or 3, **characterised in that** each hook (18, 218) is welded to the mesh (12) at its connection end.

5. Prosthesis according to Claim 2 or 3, **characterised in that** each hook (118) is extended at its connection end by a strand (120) twisted around the mesh (12).

6. Prosthesis according to any of the preceding claims, **characterised in that** each hook (18) of a single clip has at its catching end a crook shape (24), the two hooks (18) overlapping at least partially to form the said clip (16).

7. Prosthesis according to any of claims 1 to 5, **characterised in that** each hook (218) has a substantially rectilinear blade shape, the two hooks (218) running opposite each other and apart from each other when the clip is open.

8. Prosthesis according to any of the preceding claims, **characterised in that** the mesh (12) is elastically deformable towards its expanded position.

9. Blood vessel treatment kit, **characterised in that** it comprises:
- a prosthesis according to Claims 2 and 8,
- means (30) of keeping the mesh retracted in the region of the or each clip,
- a mesh releasing tube (32) defining a conduit for confining the prosthesis in its retracted state.

10. Kit according to Claim 9, **characterised in that** the said confining conduit of the releasing tube (32) comprises longitudinal channels (33) for receiving the hooks (18).

## Patentansprüche

1. Rohförmige, radial verformbare Prothese (10), umfassend ein zwischen einem zusammengezogenen Zustand mit vermindertem Durchmesser und einem erweiterten Zustand mit größeren Durchmesser verformbares Geflecht (12) und mindestens zwei von dem Geflecht (12) getragene äußere Haken (18; 118; 218), **dadurch gekennzeichnet, dass** die beiden Haken (18; 118: 218) miteinander einen Kneifer (16) zum Einhaken in ein außen liegendes Gewebe bilden, und die beiden Haken zwischen einer beabstandeten Position in welcher der Kneifers geöffnet ist und einer angenäherten Position in welcher der Kneifer geschlossen ist, beweglich sind.

2. Prothese nach Anspruch 1, **dadurch gekennzeichnet, dass** jeder Haken (18; 118; 218) an das Geflecht (12) an einem Verknüpfungsende angeknüpft ist und die Haken (18; 118; 218) eines gleichen Kneifers (16) bei der Verformung der Prothese gegeneinander beweglich sind.

3. Prothese nach Anspruch 2, **dadurch gekennzeichnet, dass** das Geflecht (12) sich kreuzende Fäden umfasst, um ein Netzwerk von verformbaren unregelmäßigen Vierecken zu bilden, und **dadurch**, dass jeder Haken (18; 118; 218) an das Geflecht in einer Ecke des Vierecks angeknüpft ist.

4. Prothese nach Anspruch 2 oder 3, **dadurch gekennzeichnet, dass** jeder Haken (18; 218) an das Geflecht (12) an seinem Verknüpfungsende angeschweißt ist.

5. Prothese nach Anspruch 2 oder 3, **dadurch gekennzeichnet, dass** jeder Haken (118) an seinem Verknüpfungsende durch ein um das Geflecht (12) geschlungenes Strangstück (120) verlängert ist.

6. Prothese nach einem der vorhergehenden Ansprüche, **dadurch gekennzeichnet, dass** jeder Haken (18) eines selben Kneifers an seinem Verknüpfungsende eine Traversenform (24) aufweist, wobei die beiden Haken (18) mindestens zum Teil übereinander greifen, um den Kneifer (16) zu bilden.

7. Prothese nach einem der Ansprüche 1 bis 5, **dadurch gekennzeichnet, dass** jeder Haken (218) eine im Wesentlichen gradlinige dünne Streifenform bildet, wobei die beiden Haken (218) aufeinander zulaufen und voneinander weg, wenn der Kneifer geöffnet wird.

8. Prothese nach einem der vorhergehenden Ansprüche, **dadurch gekennzeichnet, dass** das Geflecht (12) in Richtung seiner erweiterten Position elastisch verformbar ist.

9. Behandlungskit für ein Blutgefäß, **dadurch gekennzeichnet, dass** es umfasst:
- eine Prothese nach den Ansprüchen 2 und 8;
- Mittel (30) zur Retention des zusammengezogenen Geflechts in der Region von dem oder von jedem Kneifer;
- ein Rohr (32) zum Abwurf des Geflechts, das eine Begrenzungsleitung der Prothese in ihren zurückgezogenen Zustand festlegt.

10. Kit nach Anspruch 9, **dadurch gekennzeichnet, dass** die Begrenzungsleitung des Abwurfrohres (32) Längsrinnen (33) zur Aufnahme der Haken (18) umfasst.
